(19) Europäisches Patentamt / European Patent Office / Office européen des brevets

(11) EP 4 552 735 A2

(12) **EUROPEAN PATENT APPLICATION**

(43) Date of publication:
**14.05.2025 Bulletin 2025/20**

(21) Application number: **24206249.5**

(22) Date of filing: **11.10.2024**

(51) International Patent Classification (IPC):
***B01J 20/22*** *(2006.01)* ***B01D 53/04*** *(2006.01)*
***B01J 20/34*** *(2006.01)*

(52) Cooperative Patent Classification (CPC):
**B01J 20/226; B01D 53/0438; B01J 20/3425; B01J 20/3458**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.11.2023 JP 2023190573**

(71) Applicants:
- **L'AIR LIQUIDE, SOCIETE ANONYME POUR L'ETUDE ET L'EXPLOITATION DES PROCEDES GEORGES CLAUDE**
  **75007 Paris (FR)**
- **Kyoto University**
  **Kyoto-shi, Kyoto 606-8501 (JP)**

(72) Inventors:
- **LAVENN, Christophe**
  **78354 Les Loges-En-Josas (FR)**
- **BENZAQUI, Marvin**
  **78350 Les Loges-En-Josas (FR)**
- **OGAWA, Tomofumi**
  **Yokosuka, 239-0847 (JP)**
- **PROST, Laurent**
  **60439 Frankfurt am Main (DE)**
- **Kitagawa, Susumu**
  **Kyoto, 606-8501 (JP)**
- **Otake, Ken-ichi**
  **Kyoto, 606-8501 (JP)**

(74) Representative: **Air Liquide**
**L'Air Liquide S.A.**
**Direction de la Propriété Intellectuelle**
**75, Quai d'Orsay**
**75321 Paris Cedex 07 (FR)**

# (54) GAS SEPARATION SYSTEM, GAS SEPARATION METHOD, AND GAS SEPARATION AGENT

(57) [Problem] To provide a gas separation system, a gas separation method, and a gas separation agent with which it is possible to remove a solvent gas having oxygen atoms from a gas including a hydrocarbon gas down to a concentration of 500 ppm or lower, and that can regenerate at a low temperature of 150°C or lower under a purge gas flow or vacuum.

[Solution] A gas separation system for separating a solvent gas having oxygen atoms from a gas including a hydrocarbon gas, the gas separation system comprising: a gas separation agent; and a control unit that controls at least one among at least the flow rate, pressure, and temperature of the gas, wherein the gas separation agent has at least two organometallic complexes having a three-dimensional lattice structure defined by opposing metal-containing planar ligands, and columnar ligands coordinated between the planar ligands, and the at least two organometallic complexes form an inter-penetrating structure such that one apex portion of a unit cell of one of the organometallic complexes is positioned in a space inside one unit cell of the other organometallic complex.

[FIG. 1]

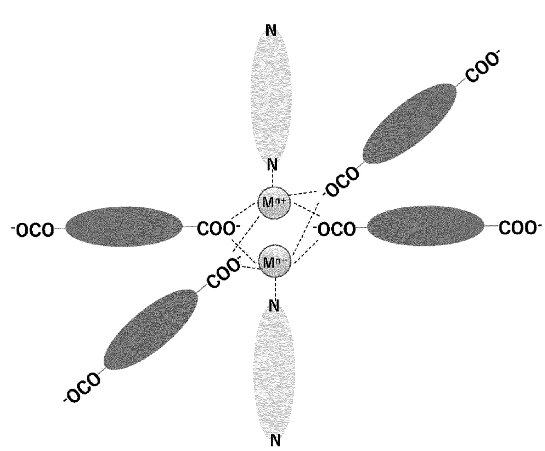

EP 4 552 735 A2

## Description

[Technical Field]

The present invention relates to a gas separation system, a gas separation method, and a gas separation agent.

[Background Art]

In contrast to other hydrocarbon gases, acetylene cannot be safely compressed at a pressure higher than 1.5 bar due to risks related to decomposition and safety. In current storage technology that uses an acetylene cylinder, acetylene is dissolved in an organic solvent (typically acetone or dimethylformamide (DMF)) permeated in a porous material (a porous calcium silicate-based material). As a result, the organic solvent unavoidably remains in the discharged acetylene during recovery of the acetylene.

Usages such as vacuum carburization (also reported as low pressure carburization or low pressure cementing), atomic absorption spectroscopy, semiconductor manufacturing, and carbon nanotube manufacturing require control and management of solvent contamination levels in order to achieve the desired quality of the process. This type of management can be performed by, for example, limiting the exhaust flow of acetylene, proper dimensioning of the process and piping, limiting the utilization of the cylinder being taken into consideration (limiting a lower residual pressure of the cylinder), or using an inline purifier. This type of purifier is typically based on a cold trap or adsorbent bed (activated carbon, zeolite) (U. S. Patent No. 8398747, WO 2008/120160).

[Prior Art Documents]

[Patent Documents]

[Patent Document 1] US 8398747 A
[Patent Document 1] WO 2008/120160 A1

[Summary of the Invention]

[Problems to be Solved by the Invention]

However, when limiting the utilization/minimum residual pressure, the utilization of the acetylene cylinder is generally limited to a specific residual pressure threshold value (0.5 MPa ~1 MPa) in order to avoid a large amount of solvent contamination. As a result, only a portion (40%~75%) of the supplied acetylene is used. The limitations on usage are difficult to apply to systems requiring a higher purity than the initially unavoidable cylinder content (approximately 300 ppm in DMF, and approximately 1.5% in acetone). In a cold trap, application is directly limited by the solvent vapour pressure of the solvent at the temperature of the cooling medium. The energy requirement depends on the constant operation of a heat exchanger and cooling system, and thus is typically high. In an activated carbon bed system, a regeneration process or phase after the adsorbent bed is saturated with captured compounds is necessary. In this step, the adsorbent bed is typically processed by vacuum or gas flow under a heating condition of a temperature from 200°C to 350°C. The regeneration of an activated carbon-based purifier requires high heat and heat energy in addition to a large amount of gas (purge gas) or vacuum over a long period of time. This adds time to the regeneration and maintenance steps, and results in an energy intensive process.

The purpose of the present invention is to provide a gas separation system, a gas separation method, and a gas separation agent with which it is possible to remove a solvent gas having oxygen atoms from a gas including a hydrocarbon gas down to a concentration of 500 ppm or lower, and that can be regenerated at a low temperature of 150°C or lower under a purge gas flow or vacuum.

[Means for Solving the Problems]

As a result of diligent study, the inventors of the present application discovered that the above-mentioned purpose can be achieved by adopting the following configuration and thus completed the present invention.

According to one embodiment, the present invention relates to

- a gas separation system for separating a solvent gas having oxygen atoms from a gas including a hydrocarbon gas,

the gas separation system comprising:

- a gas separation agent; and
- a control unit that controls at least one among at least the flow rate, pressure, and temperature of the gas, wherein
- the gas separation agent has at least two organometallic complexes having a three-dimensional lattice structure defined by opposing metal-containing planar ligands, and columnar ligands coordinated between the planar ligands, and
- the at least two organometallic complexes form an inter-penetrating structure such that one apex portion of a unit cell of one of the organometallic complexes is positioned in a space inside one unit cell of the other organometallic complex.

According to one embodiment, the planar ligand is preferably represented by at least one among formulae (1a) to (1g) below.

[CHEMICAL FORMULA 1]

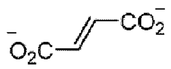
(1a)

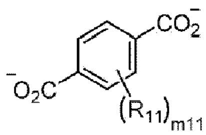
(1b)

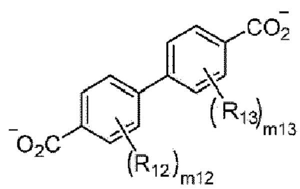
(1c)

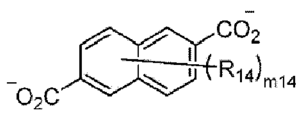
(1d)

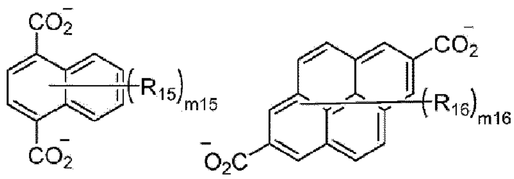
(1e) (1f)

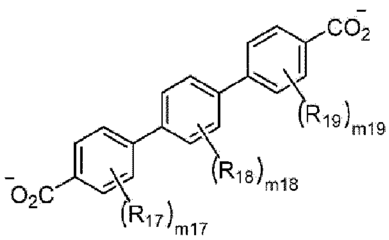
(1g)

(In the formula, $R_{11}$ to $R_{19}$ are each independently an alkyl group, an alkoxy group, a hydroxy group, a halogen atom, an alkanoyl group, a hydroxyalkyl group, a phenyl group, a phenoxy, benzyl, phenethyl, carboxy group, a cyano group, or a nitro group. If there are a plurality of each of $R_{11}$ to $R_{19}$, these may be the same or different.
m11 to m13 and m17 to m19 are each independently an integer of 0 to 4. m14 to m16 are each independently an integer of 0 to 6.)

According to one embodiment, the columnar ligand is preferably represented by at least one among formulae (2a) to (2e) below.

[CHEMICAL FORMULA 2]

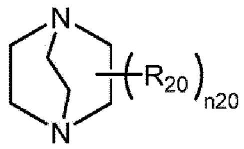

(2a)

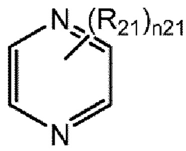

(2b)

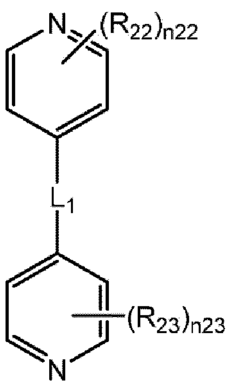

(2c)

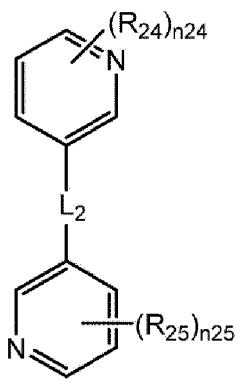

(2d)

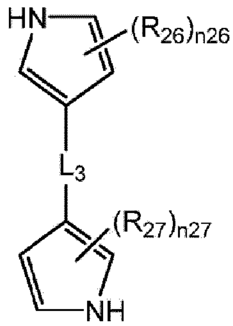

(2e)

(In the formula, $R_{20}$ to $R_{27}$ are each independently an alkyl group, an alkoxy group, a hydroxy group, a halogen atom, an alkanoyl group, a hydroxyalkyl group, a phenyl group, a phenoxy, benzyl, phenethyl, carboxyl group, a cyano group, or a nitro group. If there are a plurality of each of $R_{20}$ to $R_{27}$, these may be the same or different.

$L_1$ to $L_3$ each independently indicate a divalent linking group including a single bond or unsaturated bond.
n20 to n25 are each independently an integer of 0 to 4. n26 and n27 are each independently an integer of 0 to 3.)

According to one embodiment, the metal is preferably at least one selected from the group consisting of Sc, Ti, V, Cr, Mn, Fe, Co, Ni, Cu, Al, and Zn.
According to one embodiment, the hydrocarbon gas is preferably at least one selected from the group consisting of methane, ethane, ethylene, and acetylene.
According to one embodiment, the solvent gas is preferably at least one selected from the group consisting of acetone, dimethylformamide, diethylformamide, and water.
According to one embodiment, the control unit preferably executes

- an adsorption process for circulating the gas, and bringing the solvent gas to be separated and the gas separation agent into contact with each other; and
- a desorption process for performing decompression, heating, circulation of the purge gas that does not include the solvent gas, or a combination of these such that the adsorbed solvent gas is desorbed from the gas separation agent.

According to one embodiment, the present invention relates to

- a gas separation method for separating a solvent gas having oxygen atoms from a gas including a hydrocarbon gas, the gas separation method comprising:
- a step for preparing a gas separation agent; and

- an adsorption step for circulating the gas, and bringing the solvent gas to be separated and the gas separation agent into contact with each other, wherein
  the gas separation agent has at least two organometallic complexes having a three-dimensional lattice structure defined by opposing metal-containing planar ligands, and columnar ligands coordinated between the planar ligands, and
- the at least two organometallic complexes form an inter-penetrating structure such that one apex portion of a unit cell of one of the organometallic complexes is positioned in a space inside one unit cell of the other organometallic complex.

According to one embodiment, the gas separation method preferably further includes, after the adsorption step, a desorption step for performing decompression, heating, circulation of the purge gas that does not include the solvent gas, or a combination of these such that the adsorbed solvent gas is desorbed from the gas separation agent.

According to one embodiment, the present invention relates to

- a gas separation agent for separating a solvent gas having oxygen atoms from a gas including a hydrocarbon gas, the gas separation agent comprising
- at least two organometallic complexes having a three-dimensional lattice structure defined by opposing metal-containing planar ligands, and columnar ligands coordinated between the planar ligands, wherein

the at least two organometallic complexes form an inter-penetrating structure such that one apex portion of a unit cell of one of the organometallic complexes is positioned in a space inside one unit cell of the other organometallic complex.

[Effects of the Invention]

According to the gas separation system, gas separation method, and gas separation agent of the present invention, it is possible to remove solvent gas having oxygen atoms from a gas containing a hydrocarbon gas down to a concentration of 500 ppm or lower, to be regenerated at a low temperature of 150°C or lower under a purge gas flow or vacuum, and to achieve efficient gas separation.

[Brief Description of the Drawings]

FIG. 1 is a schematic view showing one portion of an organometallic complex of the present embodiment belonging to a metal organic framework (MOF).

FIG. 2 is an explanatory view schematically showing one example of a gas separation system according to the present embodiment.

FIG. 3 is a graph of an acetone vapour adsorption/desorption isotherm at 298 K.

FIG. 4 is TG analysis results of a stepwise heating program showing the content of a solvent gas (acetone) removed after maintaining a temperature for 1 hour.

FIG. 5 is TG analysis results of a stepwise heating program showing the content of a solvent gas (DMF) removed after maintaining a temperature for 1 hour.

FIG. 6 is XRD analysis results when acetone-exposed MOF-1 (Zn-CAT) is processed at each temperature and $N_2$ flow.

FIG. 7 is XRD analysis results when acetone-exposed MOF-2 (Cu/Zn-CAT) is processed at each temperature and $N_2$ flow.

[Description of Embodiments]

An embodiment of the present invention will be described hereinafter with reference to the drawings. The embodiment described below describes an example of the present invention. The present invention is not in any way limited to the following embodiment, and includes various modifications within a scope that does not alter the essence of the present invention. It should be noted that not all of the configurations described below are essential configurations of the present invention. In some or all of the drawings, parts that are not required for the explanations may be omitted, and parts may be expanded or reduced in scale in order to facilitate the explanation.

<Gas Separation System and Gas Separation Method>

A gas separation system of the present embodiment comprises a gas separation agent, and a control unit that controls at least on among at least the flow rate, pressure, and temperature of the gas.

(Gas Separation Agent)

The gas separation agent is a compound having at least two organometallic complexes. In one preferable embodiment, the gas separation agent is crystalline. The organometallic complex has a three-dimensional lattice structure defined by opposing metal-containing planar ligands, and columnar ligands coordinated between the planar ligands. The at least two organometallic complexes form an inter-penetrating structure such that one apex portion of a unit cell of one organometallic complex is positioned in a space inside one unit cell of the other organometallic complex.

This type of gas separation agent belongs to metal-organic frameworks (MOFs), which are also known as porous coordination polymers (PCPs). Metal-organic frameworks are a type of organic-inorganic hybrid material comprising metal ion-based nodes that form a framework through coordination bonds with a variety of organic ligands. This type of material belongs to the family of double-connected MOFs (CAT). CAT is a MOF formed from two or more independent three-dimensional trapezoidal frameworks having structures that inter-penetrate each other.

FIG. 1 is a schematic view showing one portion of an organometallic complex of the present embodiment belonging to a metal organic framework (MOF). In FIG. 1, dicarboxylic acid ligands are used as the planar ligands, and nitrogen-containing bivalent ligands are used as the columnar ligands. The carboxylic acid ions of the dicarboxylic acid ligands are planarly coordinated to two metal atoms (metal ions) to form planar lattice structures. Furthermore, the nitrogen-containing bivalent ligands are coordinated to the metal atoms so as to bridge the planar lattice structures from an axial direction (direction perpendicular to the plane). The planar lattice structures are layered, thereby forming a three-dimensional lattice structure having continuous unit cells in which the metal atoms are the apex points and the planar ligands and columnar ligands are the sides. In one preferable embodiment, the three-dimensional lattice structure is cubic.

Organometallic complexes having an inter-penetrating structure are generally formed by a procedure in which a planar lattice structure (xy plane) is formed by metal atoms and planar ligands, and the planar lattice structure is then layered in the thickness direction (z-axis direction) with columnar ligands.

However, the method for forming the organometallic complex is not limited to this. Although not particularly limited, the inter-penetration degree is preferably 2~4, more preferably 2 or 3, and even more preferably 2.

Planar ligands of the same type or different types may be used as the planar ligands. Similarly, columnar ligands of the same type or different types may be used as the columnar ligands, and metal of the same type or different types may be used as the metal. The number of metal atoms at the apex points of the unit cells can be appropriately set in accordance with the type of metal or the valence of the ions. The number of metal atoms is typically 2, 3, or 4, preferably 2 or 3, and more preferably 4.

(Metal)

A fourth period transition metal is preferably as the metal included in (coordinated to) the planar ligands. The metal is more preferably at least one selected from the group consisting of Sc, Ti, V, Cr, Mn, Fe, Co, Ni, Cu, Al, and Zn, and even more preferably at least one type selected from the group consisting of Cu, Cr, Mn, Fe, Co, Ni, Al, and Zn.

(Planar Ligand)

An aromatic polycarboxylic acid ligand is preferable as the planar ligand. An aromatic polycarboxylic acid ligand that is preferable as the planar ligand is a compound in which two or more, preferably two $COO^-$ groups are bonded to aromatic rings at positions which are line symmetrical or point symmetrical (the para position in the case of benzene rings) as preferable positions. Preferable examples of the aromatic ring include an aromatic hydrocarbon ring and an aromatic heterocyclic ring. Examples of the aromatic hydrocarbon ring include a benzene ring, naphthalene ring, anthracene ring, pyrene ring, biphenyl ring, terphenyl ring, etc. Examples of the aromatic heterocyclic ring include an imidazole ring, pyridine ring, etc. Two or more of the aromatic rings may be connected by a group such as CO, O, $CH_2$, etc.

The COOH group ($COO^-$ group) of the aromatic polycarboxylic acid ligand may be directly bonded to the aromatic ring, or may be bonded via a suitable linker (spacer) such as $-CH_2-$, - CO-, -CH(OH)-, $-CH_2CH_2-$, etc. In a preferable aromatic polycarboxylic acid ligand, the COOH group ($COO^-$ group) is directly bonded to the aromatic ring.

In addition to an aromatic polycarboxylic acid ligand, it is possible to also use an aliphatic polycarboxylic acid ligand as the planar ligand. The aliphatic polycarboxylic acid ligand is a compound in which two or more, preferably two $COO^-$ groups are bonded to aliphatic hydrocarbons at positions which are line symmetrical or point symmetrical as preferable positions. A chain aliphatic hydrocarbon is preferable as the aliphatic hydrocarbon, and a chain aliphatic

unsaturated hydrocarbon is more preferable.

The planar ligand is preferably represented by at least one among formulae (1a) to (1g) below.

[CHEMICAL FORMULA 3]

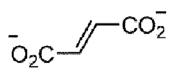

(1a)

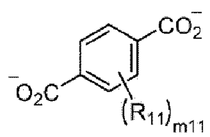

(1b)

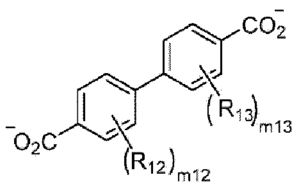

(1c)

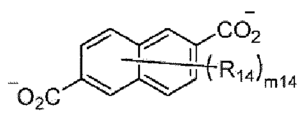

(1d)

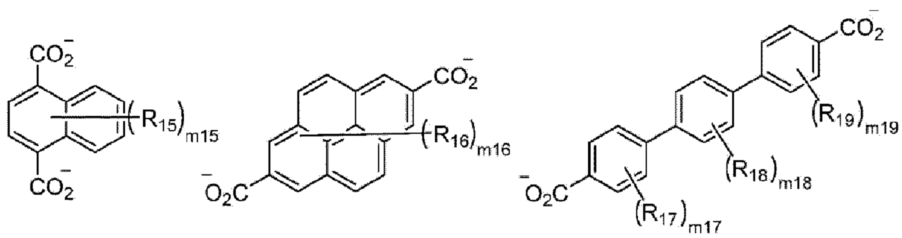

(1e) (1f) (1g)

(In the formula, $R_{11}$ to $R_{19}$ are each independently an alkyl group, an alkoxy group, a hydroxy group, a halogen atom, an alkanoyl group, a hydroxyalkyl group, a phenyl group, a phenoxy, benzyl, phenethyl, carboxy group, a cyano group, or a nitro group. If there are a plurality of each of $R_{11}$ to $R_{19}$, these may be the same or different. m11 to m13 and m17 to m19 are each independently an integer of 0 to 4. m14 to m16 are each independently an integer of 0 to 6.)

Specific examples of the alkyl group include alkyl groups having a straight chain or branch with C1 to C4, such as a methyl group, an ethyl group, an n-propyl group, an isopropyl group, an n-butyl group, a sec-butyl group, a tert-butyl group, etc.

Examples of the alkoxy group include alkoxy groups having a straight chain or branch with C1 to C4, such as a methoxy group, an ethoxy group, an n-propoxy group, an isopropoxy group, an n-butoxy group, a sec-butoxy group, a tert-butoxy group, etc.

Examples of the halogen atom include a fluorine atom, a chlorine atom, a bromine atom, and an iodine atom.

Examples of the alkanoyl group include alkanoyl groups having a straight chain or branch with C1 to C5, such as a formyl group, an acetyl group, a propionyl group, a butyryl group, a pivaloyl group, etc.

The hydroxyalkyl group indicates a group having 1~3, preferably 1~2, and particularly 1 hydrogen atom of an alkyl group substituted by an OH group.

Specific examples of the planar ligand include the structure represented by the formula below. The abbreviations in each formula are the same throughout the present specification.

[CHEMICAL FORMULA 4]

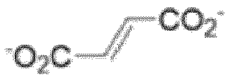
(fma)

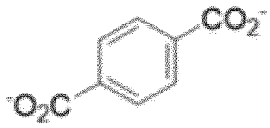
(bdc)

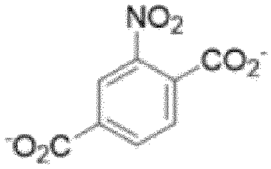
($NO_2$-bdc)

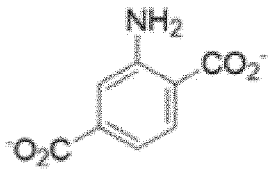
($NH_2$-bdc)

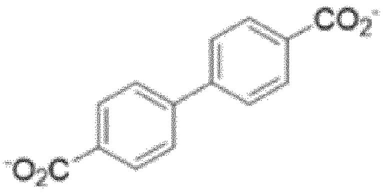
(bpdc)

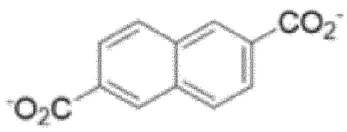
(nda)

(Columnar Ligand)

A nitrogen-containing or phosphorous-containing aromatic divalent ligand is preferable as the columnar ligand. Preferable examples of the nitrogen-containing or phosphorous-containing aromatic divalent ligand include a ligand having a nitrogen-containing or phosphorous-containing aromatic ring in which two nitrogen atoms or phosphorous atoms inside the aromatic ring are at substantially point-symmetrical positions within the molecule, and include, for example, 4,4'-bipyridyl, 3,3'-bipyridyl, pyrazine, etc. The nitrogen-containing or phosphorous-containing divalent ligand may have any spacer group inserted between two groups as long as the orientation of the coordinated two nitrogen atoms of the aromatic rings take substantially point-symmetrical positions to each other. A nitrogen-containing aromatic divalent ligand is preferable as the columnar ligand.

It is possible to also use an aliphatic heterocyclic divalent ligand as the columnar ligand. Preferable examples of the aliphatic heterocyclic divalent ligand include a ligand having a nitrogen-containing or phosphorous-containing aliphatic heterocyclic ring in which two nitrogen atoms or phosphorous atoms inside the aliphatic heterocyclic ring are at substantially point-symmetrical positions within the molecule.

The planar ligand is preferably represented by at least one among formulae (2a) to (2e) below.

[CHEMICAL FORMULA 5]

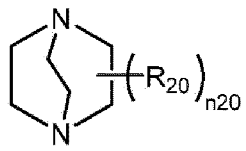

(2a)

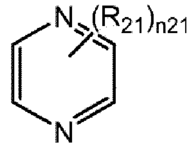

(2b)

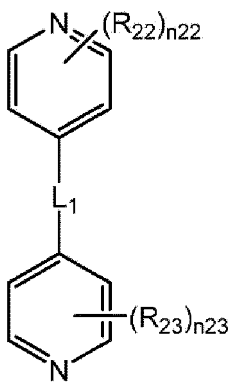

(2c)

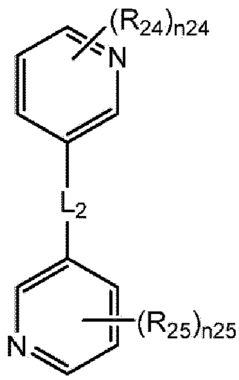

(2d)

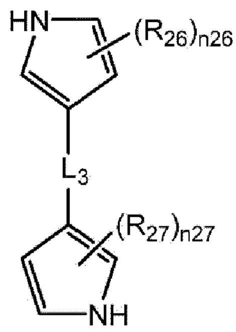

(2e)

(In the formula, $R_{20}$ to $R_{27}$ are each independently an alkyl group, an alkoxy group, a hydroxy group, a halogen atom, an alkanoyl group, a hydroxyalkyl group, a phenyl group, a phenoxy, benzyl, phenethyl, carboxyl group, a cyano group, or a nitro group. If there are a plurality of each of $R_{20}$ to $R_{27}$, these may be the same or different.

$L_1$ to $L_3$ each independently indicate a divalent linking group including a single bond or unsaturated bond.
n20 to n25 are each independently an integer of 0 to 4. n26 and n27 are each independently an integer of 0 to 3.)

Corresponding groups in $R_{11}$ to $R_{19}$ of formula (1a) to (1g) above can be preferably adopted as the alkyl group, alkoxy group, halogen atom, alkanoyl group, and hydroxyalkyl group represented by $R_{20}$ to $R_{27}$.

$L_1$ to $L_3$ each independently indicate a divalent linking group including a single bond or unsaturated bond. Examples of the divalent linking group including the unsaturated bond include a group having a double bond such as -N=N- (trans type is preferable), -CH=CH-, - CH=CH-CH=CH- or -CH=CH-CH=CH-CH=CH- (however, the double bond is preferably a trans type), a group having a triple bond such as -C≡C-, -C≡C-C≡C-, -C≡C-C≡C-C≡C-, a group represented by formulae (11) and (12) below, etc.

[CHEMICAL FORMULA 6]

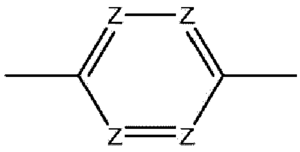

(11)

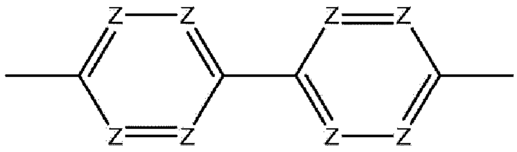

(12)

(In the formula, Z represents $CR_8$ or N. $R_8$ is an a hydrogen atom, an alkyl group, an alkoxy group, a hydroxy group, a halogen atom, an alkanoyl group, a hydroxyalkyl group, a phenyl group, a phenoxy group, a benzyl group, a phenethyl group, a carboxyl group, a cyano group, or a nitro group.)

Corresponding groups in $R_{11}$ to $R_{19}$ of formula (1a) to (1g) above can be preferably adopted as the alkyl group, alkoxy group, halogen atom, alkanoyl group, and hydroxyalkyl group represented by $R_8$.

Specific examples of the column ligand include the structure represented by the formula below. Examples include the structure represented by the formula below. The abbreviations in each formula are the same throughout the present specification.

[CHEMICAL FORMULA 7]

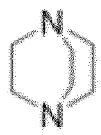

(dabco)

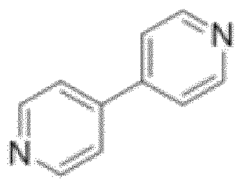

(bpy)

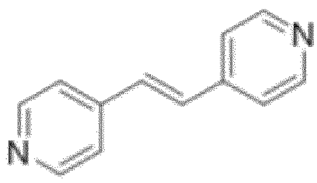

(dpe)

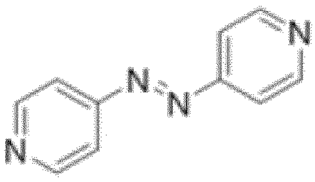

(azpy)

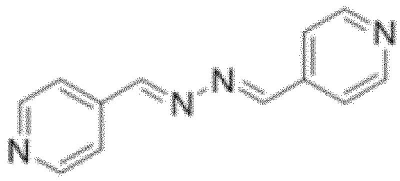

(bpmh)

In the gas separation system, an organometallic complex is used as the gas separation (adsorption) agent. The organometallic complex has a three-dimensional lattice structure defined by opposing metal-containing planar ligands, and columnar ligands coordinated between the planar ligands, and forms an inter-penetrating structure. The size or shape of the three-dimensional lattice structure can be modified in accordance with the size (in particular, length) of the columnar ligands coordinated between the planar ligands. In the gas separation system, the adsorption of the solvent gas onto the organometallic complex or desorption is controlled and promoted, thereby making it possible to perform gas separation efficiently. The reason for this is not certain, but the following is inferred. First, the adsorption agent can take both an "open" phase and a "closed" phase. The first step corresponds to the opening of the MOF. This step is performed by initial adsorption of a hydrocarbon gas (including a certain degree of a solvent gas) under initial conditions (initialization) for which the pressure/temperature conditions of the hydrocarbon gas are known to cause a gate opening phenomenon. Exchange occurs between the solvent gas and the hydrocarbon gas while the gas separation agent is in the open state during the in-line purification step. In this type of exchange, the interaction between the MOF and solvent gas is greater

than the interaction between the MOF and hydrocarbon gas, and thus this type of exchange works advantageously for the apparent trapping of acetylene within the pores of the gas separation agent. This change in affinity can be confirmed by the gate opening pressure. If assuming that the MOF phase is similar in the closed phase and the open phase for both the solvent gas and hydrocarbon gas in practice, then the corresponding change in energy depends only on the interaction between the substrate and the guest. Therefore, a low pressure of the gate opening part observed in the case of solvent gas adsorption (when compared to hydrocarbon gas adsorption at the same temperature) indicates that the affinity between the MOF and solvent gas is superior to the hydrocarbon gas.

Meanwhile, the gas separation agent has an organometallic complex exhibiting a highly flexible inter-penetrating structure. This makes it possible to easily and efficiently desorb the solvent gas to regenerate the gas separation agent by merely performing a purge gas flow, decompression, low temperature heating, or a combination of these.

(Method for Manufacturing Gas Separation Agent)

The method for manufacturing the gas separation agent is not particularly limited and can include methods that are well known in the manufacturing of MOFs. Specific examples include a one-pot synthesis method (e.g., self-assembling method, solvothermal method, microwave irradiation method, ionothermal method, high throughput method, etc.), a gradual synthesis method (e.g., metal-organic nodule structure precursor complex method, complex ligand method, in-situ sequential synthesis method, post-synthesis modification method, etc.), sonochemical synthesis method, mechanochemical synthesis method, etc.

Manufacturing examples adopting the self-assembling method, i.e., one of the one-pot synthesis methods, include the following method. For example, a metal salt (e.g., metal nitrate, etc.) that provides a metal centre, and planar ligands that provide a planar lattice structure are mixed in a solvent. A mixture containing columnar ligands and a solvent is added to the mixture containing the complex having the planar lattice structure, and this mixture is reacted under room temperature or heating. This makes it possible to manufacture a gas separation agent with inter-penetrating cubic lattice organometallic complexes.

Examples of solvents that can be used for dissolving the ligands or metal salt include, but are not particularly limited to, cyclic or acyclic amide-based solvents such as dimethylformamide (DMF) or N-methylpyrrolidone, alcohol-based solvents such as methanol or ethanol, ketone-based solvents such as acetone, and aromatic-based solvents such as toluene, and water. The reaction temperatures are preferably 25°C to 150°C, and more preferably 70°C to 130°C. The reaction time is preferably 2 hours to 72 hours, and more preferably 6 hours to 48 hours. The target gas separation agent can be manufactured by collecting the product of the reaction by filtration or centrifugation, for example, washing the product with the solvent noted above as necessary, and then drying the product.

(Control Unit)

A control unit not shown in the drawings controls at least one among at least the flow rate, pressure, and temperature of the gas. Known computing devices such as CPUs or MPUs can be used as the control unit.

In the gas separation system of the present embodiment, the control unit preferably executes: an adsorption process for circulating the gas, and bringing the solvent gas to be separated and the gas separation agent into contact with each other; and a desorption process for performing decompression, heating, circulation of the purge gas that does not include the solvent gas, or a combination of these such that the adsorbed solvent gas is desorbed from the gas separation agent. An organometallic complex exhibiting both a molecular sieve effect and a chemical sieve effect is used to control and promote adsorption of the solvent gas onto the organometallic complex and desorption by control of the flow rate of gas, etc., thereby making it possible to perform gas separation at a comparatively low pressure and low temperature.

(Gas Separation Method)

A gas separation method of the present embodiment includes a step for preparing a gas separation agent, and an adsorption step for circulating the gas and bringing the solvent gas to be separated and the gas separation agent into contact with each other. Furthermore, the gas separation method of the present embodiment includes, after the adsorption step, a desorption step for performing decompression, heating, circulation of the purge gas that does not include the solvent gas, or a combination of these such that the adsorbed solvent gas is desorbed from the gas separation agent. The aforementioned gas separation agent can be used as the gas separation agent, and thus the adsorption step and the desorption step will be described below. FIG. 2 is an explanatory view schematically showing one example of a gas separation system according to the present embodiment. The present embodiment provides an in-line purification system for capturing/removing a solvent gas from a gas flow. The gas separation method will be described while referring to FIG. 2.

A gas separation system 20 comprises a gas supply port 1, a gas separation agent 4, and a gas discharge port 7. The gas supply port 1, the gas separation agent 4, and the gas discharge port 7 are connected by a tube. A column (not

shown in the drawings) filled with the gas separation agent 4 is provided with a heater 5 for heating the column. A pressure control unit 2 and a mass flow controller 3 are interposed between the gas supply port 1 and the gas separation agent 4, and control the flow rate and pressure of the gas. A pressure gauge 9 is connected between the mass flow controller 3 and the gas separation agent 4. A detector 6 is interposed between the gas separation agent 4 and the gas discharge port 7, and detects the concentration of the solvent gas, etc. A purge gas source 8 is connected between the pressure control unit 2 and the mass flow controller 3, and supplies a purge gas. A purge gas source 10 is connected between the gas separation agent 4 and the detector 6. The flow rate of the purge gas source 10 may be controlled by the mass flow controller 11.

(Adsorption Step)

The gas supply port 1 is typically an acetylene cylinder. The gas from the gas supply port 1 is an acetylene stream that contains acetylene as a hydrocarbon gas, and contains acetone or DMF as a solvent gas. The concentration of the acetone is 0.5% ~ 30% by volume, and the concentration of DMF is 100 ppm~7000 ppm.

The hydrocarbon gas is preferably at least one among methane, ethane, ethylene, and acetylene. The solvent gas is preferably at least one among acetone, dimethylformamide, diethylformamide, and water.

The gas separation agent 4 can be in the form of a powder material, compressed powder material, or extrusion. The gas separation agent 4 may be mixed with a binder to form pellets. The binder can constitute 1 wt%~25 wt% of the pellets. Examples of the binder include polybutadiene, a styrenic thermoplastic elastomer, polyvinylidene fluoride, a polyamide, a polyvinyl alcohol, or a combination of these. This type of gas separation agent 4 is filled into one or a plurality of columns. The gas circulates through the column, thereby adsorbing the solvent gas onto the gas separation agent 4 and separating the solvent gas. The size and shape of the column, and the filling and amount of the gas separation agent 4 in the one or more columns are optimized for the target usage conditions.

The flow rate of the gas is preferably 0.1 slm or higher, more preferably 1 slm or higher, and even more preferably greater than 5 slm. The pressure of the gas is preferably atmospheric pressure, and 100 mbarG or greater is preferable.

The purification process operation (exhaust of acetylene and in-line purification) can be carried out at -50°C to 50°C, preferably -10°C to 40°C, and even more preferably 0°C to 30°C, and the temperature of the gas separation system 20 can be controlled within the aforementioned temperature ranges.

By going through the adsorption step, the concentration of the DMF is preferably set to be less than 500 ppm, and the concentration of the acetone is preferably set to be less than 5000 ppm at the gas discharge port 7. The concentrations of the DMF and acetone are both even more preferably set to be less than 100 ppm.

(Desorption Step)

The desorption step can be performed by typically circulating the purge gas in the column, and depressurizing the column. Before the desorption step, the supply of gas from the gas supply port 1 may be stopped, and the purge gas from the purge gas source 8 may be supplied. Examples of the purge gas include nitrogen gas, helium gas, argon gas, acetylene gas, etc.

Circulation and depressurization of the purge gas may be repeated. The flow rate of gas is 20 sccm to 30 sccm per 1 g of the gas separation agent 4.

The temperature of the column in the desorption step is preferably 20°C to 150°C, and more preferably 50°C to 100°C. Heating of the column is performed by the heater 5. Examples of the heater 5 include a probe heater, an induction heater, and a microwave heater. A high-temperature purge gas may be circulated together with or instead of using the heater 5.

Two or more columns are provided to the gas separation system 20, and the adsorption step may be performed with one or more columns, while the desorption step may be performed with a different one or more columns.

<Gas Separation Agent>

The gas separation agent of the present embodiment is for separating a solvent gas having oxygen atoms from a gas including a hydrocarbon gas, wherein the gas separation agent has at least two organometallic complexes having a three-dimensional lattice structure defined by opposing metal-containing planar ligands, and columnar ligands coordinated between the planar ligands, and the at least two organometallic complexes form an inter-penetrating structure such that one apex portion of a unit cell of one organometallic complex is positioned in a space inside one unit cell of the other organometallic complex. A gas separation agent in the gas separation system can be preferably adopted as this type of gas separation agent.

[Examples]

The present invention will be described in greater detail using examples below, but the present invention is not limited to the following examples as long as the gist of the invention is not exceeded.

(A. General Procedure)

The gas separation system shown in FIG. 2 was used to perform the examples. A welding grade acetylene feed calibrated to have a supply pressure using acetone of 0.08 MPa was used as the solvent. The adsorption column had an internal volume of 25 mL and was filled with the gas separation agent 4. The volume of acetylene passing through the column was determined by integrating the acetylene flow rate measured by a mass flow controller (MFC) 3. The acetone concentration was calculated by comparing a spectrum from a Fourier transform infrared spectroscopy device (FTIR) 6 (Nicolet 6700, manufactured by ThermoFisher) with known standards for acetone.

The materials used in the examples and comparative examples are shown in table 1 below.

[Table 1]

| Type | Abbreviation | Material name (manufacturing company) | Remarks |
|---|---|---|---|
| Activated carbon | ACC-1 | G-BAC 700 (Manufactured by Kureha Corporation) | Petroleum pitch activated carbon |
| Activated carbon | ACC-2 | Norti(Registered trademark) RB4 (Manufactured by Cabot Corporation) | Vapor activation Extruded carbon |
| Activated carbon | ACC-3 | Norti(Registered trademark) RGM3 (Manufactured by Cabot Corporation) | Impregnation activation High skeleton density carbon |
| MOF | MDF-1 | Zn-CAT (Synthesized) | Flexible linking MOF |
| MOF | MOF-2 | Cu/Zn-CAT (Synthesized) | Flexible linking HMOF |
| MOF | MOF-3 | Zn-CAG(Synthesized) | Rigid non-linking MOF |

The properties of all synthesized MOF materials were evaluated prior to the present example using powder X-ray diffraction and a gas adsorption isotherm, and the material phases were confirmed. The completeness or solvent gas compatibility (in the case of MOF) of the materials were evaluated using gas adsorption analysis of recovered samples exposed to vapour of the solvent gas.

The materials were further evaluated by using a $BEL_{sorp-max}$ (Bel Japan, Inc.) system used in a steam mode, and using a solvent vapour adsorption isotherm. Temperature calibration was performed by a constant temperature bath. Degassing was performed three times using a $BEL_{sorp-max}$II freeze pump program using dry acetone.

The gas adsorption isotherm was equipped with a cryostat, and a volume adsorption device (BELsorp-MAX, BELsorp-mini-II) (BEL Japan, Inc.) was used, which is controlled by BEL-cryo software (BELsorp-MAX) and a bath or dewar tank for temperature control. Typically, 100 mg samples were sampled for analysis, and after all samples were degassed, adsorption measurement was performed at 423 K for at least 6 hours under a vacuum, and guest molecules (solvent gas) were removed, and reactivated between different adsorption measurements.

The materials were further evaluated by immersing the materials into a solvent, and exposure ex-sit to solvent vapour on the dried materials. After solvent gas exposure (DMF, acetone), drying of the materials was performed in air in the case of acetone, and under reduced pressure in the case of DMF.

The solvent gas exposure material was examined using thermogravimetric analysis (TGA) carried out by Rigaku Instrument TG8120. A sample of approximately 3 mg~10 mg was heated under a nitrogen flow of 200 mL ▪ $min^{-1}$ in an aluminium crucible. The analysis was started from ambient temperature at a heating rate of 5°C ▪ $min^{-1}$, and maintained for 1 hour at a specific temperature (50°C ~ 300°C), after which the temperature was increased to a maximum of 500°C at a heating speed of 5°C ▪ $min^{-1}$. Based on these analyses, the regeneration temperature was estimated by comparing weight loss of a constant end (when caused by loss of solvent gas) to the total solvent gas loss.

The phase change of a flexible MOF during solvent gas desorption was evaluated by temperature-dependent powder X-ray diffraction carried out by Rigaku MiniFlex 600C equipped with an Anton Parr BTS-500 oven. Before analysis, the flexible MOF sample was immersed in acetone or DMF. Before each analysis for temperature stabilization, diffraction patterns were collected at a holding time of two minutes with 5°C to one minute of 5 ~60° (20) at a temperature in a range of 25°C~ 130°C. XRD is collected under an $N_2$ flow
(10 ml ▪ $min^{-1}$).

(B. Chemical Substance and Substance Synthesis)

All chemical substances and solvents were purchased at commercial quality and used without further purification.

(Synthesis of Zn-CAT (MOF-1))

Zinc(II) nitrate (2 equivalents, 0.2 mol) was dissolved in minimal DMF, and added to a solution of bdc (2 equivalents, 0.2 mol) dissolved in DMF. The mixture was heated in an oil bath set to 100°C. An ethanol solution of bpy (1 equivalent, 0.1 mol) was added dropwise to the mixture. The total solvent volume was 1L, and the solvent composition was ethanol (40% by volume) and DMF (60% by volume). For approximately 5 minutes to 1 hour after adding, the reactant was stirred at 100°C (temperature control by constant temperature oil bath). After 24 hours ~ 48 hours of reaction, the reaction mixture was cooled to room temperature, the precipitate was recovered by centrifugation, and washed three times with DMF and three times with ethanol to remove unreacted species. The powder was dried for several hours under reduced pressure, and the yield was approximately 98%.

(Synthesis of Cu/Zn-CAT (MOF-2))

A bdc (2 eq) solution dissolved in minimal DMF was added to a solution of copper(II) nitrate (1.5 eq) in DMF and zinc(II) nitrate (0.5 eq) (Zn/[Zn+Cu]=25 mol%). Next, a solution of bpy (1 equivalent) in DMF was added dropwise to a mixture placed in an oil bath set to 100°C ~ 120°C. After adding, the reactant was stirred at 100°C ~ 120°C (temperature control by constant temperature oil bath). After 24 hours ~48 hours of reaction, the reaction mixture was cooled to room temperature, the precipitate was recovered by centrifugation, and washed three times with DMF and three times with ethanol to remove unreacted species. The powder was dried for several hours under reduced pressure, and the yield was approximately 90%.

Note that the final desired metal molar ratio can be controlled with favourable fitting between the Zn:Cu input molar ratio during synthesis and that which is observed on the material after synthesis.

(Synthesis of Zn-CAG (MOF-3))

Zinc(II) nitrate (2 equivalents, 0.2 mol) was dissolved in minimal DMF, and added to a solution of bdc (2 equivalents, 0.2 mol) dissolved in DMF. The mixture was heated in an oil bath set to 100°C. Next, a solution of bpy (1 equivalent, 0.1 mol) in DMF was slowly added to the mixture. The total solvent volume was 1 L. For approximately 5 minutes after adding, the reactant was stirred at 120°C (temperature control by constant temperature oil bath). After 24 hours ~48 hours of reaction, the reaction mixture was cooled to room temperature, the precipitate was recovered by centrifugation, and washed three times with DMF to remove unreacted species. The powder was dried for several hours under reduced pressure, and the yield was approximately 90%.

(Moulding of Material)

The synthetic material described above was mixed with 0 ~ 8% by weight of "Septon" (thermoplastic elastomer manufactured by Kuraray Co., Ltd.), and/or 0~8% by weight of polybutadiene, and moulded by extrusion moulding ("Caleva", manufactured by Caleva Multi Lab). The extruded product was sieved to achieve the desired size, typically 1 ~5 mm.

(C. Preparation of Column)

For all columns, around 15 g of gas separation agent pellets (see table 2 below) was introduced to a 25 cc stainless steel column. The gas separation agent was activated (initialized) under a vacuum at 150°C for 16 hours.

[Table 2]

| | Gas separation agent | Introduced amount (g) |
|---|---|---|
| Example 1 | MOF-1 | 15.2 |
| Example 2 | MOF-2 | 14.3 |
| Comparative example 1 | MOF-3 | 10.0 |
| Comparative example 2 | ACC-1 | 15.6 |

(D. Experimental Conditions)

A column filled with the gas separation agent was set up after activation, and kept at a constant temperature of 25°C using a constant temperature bath. For evaluation, acetylene gas was caused to flow to a bed at 200 standard cubic centimetres/minute (sccm) after initialization of the bed. The initialization can correspond to setting the bed to an initial state, and can correspond to loading of acetylene of a specific volume or pressure or the flow of acetylene of a specific volume during a specific period of time. The acetylene flow was integrated to determine the total volume of acetylene flowing through the gas separation agent bed. The content of solvent gas present during an output flow of the acetylene was measured by FT-IR at a fixed measurement point. The obtained results were indicated on a typical plot of the concentration of the solvent gas by volume of purified acetylene. Furthermore, the amount H of solvent gas removed from the acetylene flow (typically represented by $cc \cdot g^{-1}$) was calculated from an initial concentration $\eta_0$ of the solvent gas during the acetylene flow in accordance with formula 2. Formula 2 is a simplified version of formula 1 corresponding to a constrained pressure region (and a small acetylene volume determined or measured by using formula 3 thereafter).

During experimentation, it was assumed that the content of the solvent gas is fixed relative to the discharged acetylene volume.

[Number 1]

$$H = \frac{\int_{Pi}^{Pf} \eta(P)dP}{m_{\text{Separation agent}}} \quad \textbf{(Formula 1)}$$

$$H = \frac{\eta_0 \Delta(V_{C2H2})}{m_{\text{Separation agent}}} \quad \textbf{(Formula 2)}$$

$$V_{C2H2} = P_{C2H2} Q_s (s_{C2H2}(P_f) - s_{C2H2}(P_i)) \quad \textbf{(Formula 3)}$$

H corresponds to the conceivable contaminant removal ability of the gas separation agent, P is the residual pressure of the acetylene cylinder, $P_i$ is the initial cylinder pressure (before using a filter), $P_f$ is the final cylinder residual pressure (end of use of filter), $\eta$ corresponds to the impurity concentration (typically considered to be acetone or DMF), $\eta_0$ is the impurity concentration at the start of using the filter, $m_{\text{separation agent}}$ is the amount of the gas separation agent inside the filter, and $V_{C2H2}$ is the volume of acetylene flowing through the filter, which can be measured or estimated (typically by using formula 3). $\eta(P)$ is the function (ordinarily the empirical fitting) of the amount of solvent (or other impurities) discharged from a solvent-based system, which is dependent on the temperature T, flow rate V, properties of the solvent, and rest/use times (tr, tu), in particular, and which can be expressed as a function of residual pressure inside the cylinder under fixed conditions. Additionally, $P_{C2H2}$ is the density of the acetylene, $Q_s$ is the solvent amount in a single cylinder among a plurality of cylinders taken into consideration, and $s_{C2H2}$ is the solubility in the solvent considered as a function of pressure (which can be empirically obtained).

Nitrogen was caused to flow as a purge gas into a 220 sccm heat saturated (externally heated at a specific temperature) gas separation agent to thereby regenerate the solvent saturated gas separation agent, and the solvent gas content of the purge gas was tracked by FT-IR in order to determine the regeneration time. A constant temperature bath was used to fix the temperature of the gas separation agent to 50°C, 60°C, or 70°C. A vacuum oven was used to heat the column at 150°C under a vacuum, thereby performing further regeneration after solvent gas saturation. For clarity, unless otherwise specified, the method for regeneration refers to vacuum regeneration under heating (150°C unless otherwise specified).

[Example 1]

The gas separation agent (flexible MOF-1) filled into the column was exhausted, followed by cooling to 15°C, and pressurizing to 0.05 MPa of acetylene before reheating to 25°C, thereby performing initialization. Purification was executed at a fixed flow rate of 200 sccm at 25°C. A constant temperature bath was used to control the temperature, and the acetone concentration was monitored using FT-IR. In example 1, the observed purification time was 2.5 hours, and

this corresponds to a $C_2H_2$ capacity of 30 L ($\eta_0$: estimated 4.1%) and an acetone removal capacity of 80 cc/g at the point in time of purification completion.

[Example 2]

The gas separation agent (flexible MOF-2) filled into the column was exhausted, followed by pressurizing to 0.05 MPa of acetylene at 25°C, thereby performing initialization. Purification was executed at a fixed flow rate of 200 sccm at 25°C. A constant temperature bath was used to control the temperature, and the acetone concentration was monitored using FT-IR. In example 2, the observed purification time was 1.7 hours, and this corresponds to a $C_2H_2$ capacity of 20 L ($\eta_0$: estimated 5.5%) and an acetone removal capacity of 80 cc/g at the point in time of purification completion.

[Comparative Example 1]

The gas separation agent (flexible MOF-3) filled into the column was exhausted, followed by pressurizing to 0.05 MPa of acetylene at 25°C, thereby performing initialization. Purification was executed at a fixed flow rate of 200 sccm at 25°C. A constant temperature bath was used to control the temperature, and the acetone concentration was monitored using FT-IR. In comparative example 1, the observed purification time was approximately 1 hour, and this corresponds to a $C_2H_2$ capacity of 11 L ($\eta_0$: estimated 3.5%) and an acetone removal capacity of 39 cc/g at the point in time of purification completion.

[Comparative Example 2]

The gas separation agent (activated carbon ACC-1) filled into the column was exhausted, followed by pressurizing to 0.05 MPa of acetylene at 25°C, thereby performing initialization. Purification was executed at a fixed flow rate of 200 sccm at 25°C. A constant temperature bath was used to control the temperature, and the acetone concentration was monitored using FT-IR. In comparative example 2, the observed purification time was approximately 8.6 hours, and this corresponds to a $C_2H_2$ capacity of 103 L ($\eta_0$: estimated 2.7%) and an acetone removal capacity of 180 cc/g at the point in time of purification completion.

(E. Comparison of Gas Separation Agents)

The materials were compared using an acetone vapour adsorption isotherm executed at 298 K for MOF-1 (Zn-CAT), MOF-2 (Cu/Zn-CAT), and three activated carbon ACC-1 (G-BAC70D), ACC-2 (Norit (registered trademark) RB4), and ACC-3 (Norit (registered trademark) RGM3) used as reference materials. FIG. 3 is a graph of an acetone vapour adsorption/desorption isotherm at 298 K. The black symbols correspond to adsorption, and the white symbols correspond to desorption. Table 3 below shows the capacity (Q) of acetone and 95% desorption pressure ($P_{95\%}$) at 288K and 308K in addition to 298 K. The capacity (Q) of acetone is measured at an absolute pressure of 10 kPa and is expressed as cc/g. $P_{95\%}$ corresponds to an estimated pressure reached in order to remove 95% of the solvent gas adsorbed by the gas separation agent. A comparison at 298 K shows that the MOF-1 and MOF-2 samples have a lower capacity than activated carbon, so as to be determined by the saturation amount with acetone vapour at 10 kPa. Furthermore, the MOF-1 and MOF-2 materials exhibited complete (95% of capacity by estimation) desorption at a pressure one order of magnitude higher than the activated carbon material at the same temperature. Therefore, the results show that (i) the capacity of MOF is low, but stays in the same capacity range, and that (ii) MOF releases acetone supplemented with a considerably high pressure, and lowers the requirement for regeneration.

[Table 3]

| Material | | 288 K | | 298 K | | 308 K | |
|---|---|---|---|---|---|---|---|
| | | Q (cc/g) | $P_{95\%}$ (kPa) | Q (cc/g) | $P_{95\%}$ (kPa) | Q (cc/g) | $P_{95\%}$ (kPa) |
| MOF-1 | Zn-CAT | 76 | 0.1 | 76 | 0.3 | 75 | 0.7 |
| MOF-2 | Cu/Zn-CAT | 69 | $8 \times 10^{-2}$ | 66 | 0.18 | 65 | 0.3 |
| MOF-3 | Zn-CAG | 203 | $2 \times 10^{-2}$ | 196 | $4 \times 10^{-2}$ | 190 | $7 \times 10^{-2}$ |
| ACC-1 | G-BAC 70D | 154 | $1 \times 10^{-2}$ | 150 | $1 \times 10^{-2}$ | 144 | $6 \times 10^{-2}$ |
| ACC-2 | Norti(Registered trademark) RB4 | 112 | $8 \times 10^{-3}$ | 112 | $1 \times 10^{-2}$ | 100 | $2 \times 10^{-2}$ |
| ACC-3 | Norti(Registered trademark) RGM3 | 132 | $2 \times 10^{-2}$ | 129 | $1 \times 10^{-2}$ | 122 | $3 \times 10^{-2}$ |

(Regeneration of Column)

For regeneration of the column, MOF-1 (Zn-CAT), MOF-2 (Cu/Zn-CAT), and ACC-1 (G-BAC 70R) were selected. These were selected by taking into consideration the higher release pressure together with the higher capacity of ACC-1, as indicated by the solvent vapour adsorption. After the selected gas separation agent was saturated with the solvent gas, 50°C, 60°C, and 70°C heat was used under 200 sccm of nitrogen as a purge gas, or the gas separation agent was regenerated for one night under vacuum conditions at 150°C. The solvent gas content of the purge gas was monitored by FT-IR. The results are shown in table 4 below. In the table, the upper row shows regeneration processing time, and the lower row shows residual solvent gas (acetone) concentration.

[Table 4]

| Material | | $N_2$Flow | | | 150°C (Vacuum oven) |
|---|---|---|---|---|---|
| | | 50°C | 60°C | 70°C | |
| MOF-1 | Zn-CAT | 6 h<br>0 ppm | 3.5 h<br>0 ppm | 2.5 h<br>0ppm | 0 ppm |
| MOF-2 | Cu/Zn-CAT | - | 3.5 h<br>0 ppm | 3 h<br>0 ppm | 0ppm |
| ACC-1 | G-BAC 70D | - | - | 3h<br>2,500ppm<br>12 h<br>300 ppm | - |

Therefore, when repeatedly used, the flexible MOF exhibits the clear advantages of it being possible to recover capacity at low energy cost.

(Regeneration Evaluation by Thermogravimetric Analysis)

Thermogravimetric analysis (TGA) was executed for a fixed time (1 hour) at different temperatures. FIG. 4 is TG analysis results of a stepwise heating program showing the content of a solvent gas (acetone) removed after maintaining a temperature for 1 hour. FIG. 5 is TG analysis results of a stepwise heating program showing the content of a solvent gas (DMF) removed after maintaining a temperature for 1 hour.

Based on fig. 4 of acetone exposure, in the case of MOF-1 and MOF-2, it was observed that the temperature for regeneration with a time limit significantly decreased. MOF-3, ACC-1, and ACC-3 each exhibited the same behaviour, and a temperature of 150°C was necessary to completely regenerate within one hour. ACC-2 required the highest regeneration temperature.

Based on fig. 5 of DMF exposure, MOF-1 and MOF-2 exhibited complete regeneration behaviour in 1 hour at 100°C. MOF-3, ACC-1, and ACC-3 required a temperature of 150°C. ACC-2 required a considerably high temperature in order to completely remove the solvent gas in 1 hour.

When using a flexible MOF material, it is possible to lower the temperature and regeneration time or both required for regeneration, thereby lowering energy consumption of the system. Zn-CAG, which is rigid MOF, exhibits the same behaviour as activated carbon in terms of TG, and shows the importance of MOF flexibility.

(XRD Analysis of Acetone-exposed Sample)

FIG. 6 and FIG. 7 show XRD analysis results when acetone-exposed MOF-1 (Zn-CAT) and MOF-2 (Cu/Zn-CAT) are respectively processed at each temperature and $N_2$ flow. This analysis shows that the change in the gas separation agent at a low temperature of 50°C is caused by phase transition (flexible gas separation agent) corresponding to regeneration of the gas separation agent releasing acetone.

[Description of Reference Characters]

1 Gas supply port
2 Pressure control unit

3, 11 Mass flow controller
4 Gas separation agent
5 Heater
6 Detector
7 Gas discharge port
8, 10 Purge gas source
9 Pressure gauge
20 Gas separation system

## Claims

**1.** A gas separation system for separating a solvent gas having oxygen atoms from a gas including a hydrocarbon gas, the gas separation system comprising:

- a gas separation agent; and
- a control unit that controls at least one among at least the flow rate, pressure, and temperature of the gas, wherein
- the gas separation agent has at least two organometallic complexes having a three-dimensional lattice structure defined by opposing metal-containing planar ligands, and columnar ligands coordinated between the planar ligands, and
- the at least two organometallic complexes form an inter-penetrating structure such that one apex portion of a unit cell of one of the organometallic complexes is positioned in a space inside one unit cell of the other organometallic complex.

**2.** The gas separation system according to claim 1, wherein the planar ligand is represented by at least one among formulae (1a) to (1g) below.

[CHEMICAL FORMULA 1]

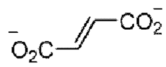

(1a)

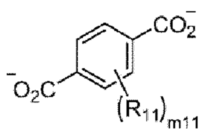

(1b)

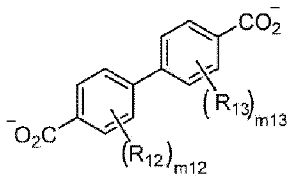

(1c)

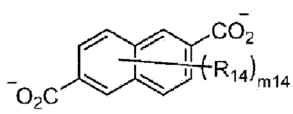

(1d)

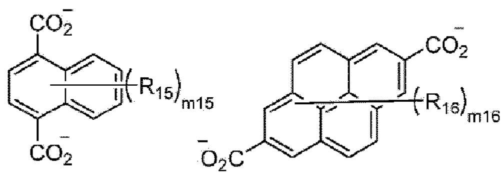

(1e) (1f)

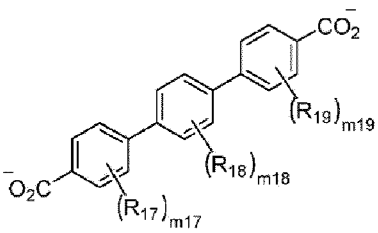

(1g)

(In the formula, $R_{11}$ to $R_{19}$ are each independently an alkyl group, an alkoxy group, a hydroxy group, a halogen atom, an alkanoyl group, a hydroxyalkyl group, a phenyl group, a phenoxy, benzyl, phenethyl, carboxy group, a cyano group, or a nitro group. If there are a plurality of each of $R_{11}$ to $R_{19}$, these may be the same or different.
m11 to m13 and m17 to m19 are each independently an integer of 0 to 4. m14 to m16 are each independently an integer of 0 to 6.)

**3.** The gas separation system according to claim 1, wherein the columnar ligand is represented by at least one among

formulae (2a) to (2e) below.

[CHEMICAL FORMULA 2]

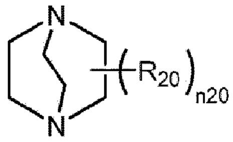

(2a)

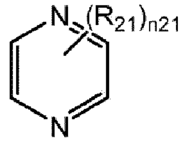

(2b)

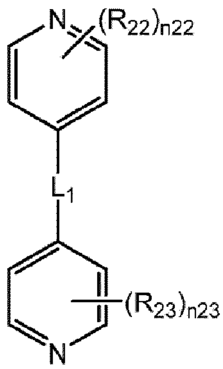

(2c)

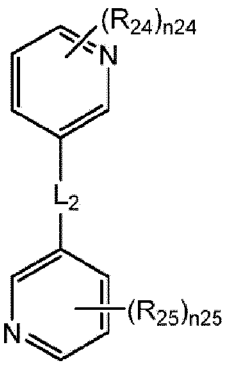

(2d)

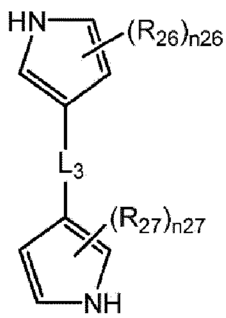

(2e)

(In the formula, $R_{20}$ to $R_{27}$ are each independently an alkyl group, an alkoxy group, a hydroxy group, a halogen atom, an alkanoyl group, a hydroxyalkyl group, a phenyl group, a phenoxy, benzyl, phenethyl, carboxyl group, a cyano group, or a nitro group. If there are a plurality of each of $R_{20}$ to $R_{27}$, these may be the same or different.

$L_1$ to $L_3$ each independently indicate a divalent linking group including a single bond or unsaturated bond. n20 to n25 are each independently an integer of 0 to 4. n26 and n27 are each independently an integer of 0 to 3.)

**4.** The gas separation system according to claim 1, wherein the metal is at least one selected from the group consisting of Sc, Ti, V, Cr, Mn, Fe, Co, Ni, Cu, Al, and Zn.

**5.** The gas separation system according to claim 1, wherein the hydrocarbon gas is at least one selected from the group consisting of methane, ethane, ethylene, and acetylene.

**6.** The gas separation system according to claim 1, wherein the solvent gas is at least one selected from the group consisting of acetone, dimethylformamide, diethylformamide, and water.

**7.** The gas separation system according to claim 1, wherein the control unit executes:

- an adsorption process for circulating the gas, and bringing the solvent gas to be separated and the gas separation agent into contact with each other; and
- a desorption process for performing decompression, heating, circulation of the purge gas that does not include the solvent gas, or a combination of these such that the adsorbed solvent gas is desorbed from the gas separation agent.

**8.** A gas separation method for separating a solvent gas having oxygen atoms from a gas including a hydrocarbon gas, the gas separation method comprising:

- a step for preparing a gas separation agent; and
- an adsorption step for circulating the gas, and bringing the solvent gas to be separated and the gas separation agent into contact with each other, wherein
- the gas separation agent has at least two organometallic complexes having a three-dimensional lattice structure defined by opposing metal-containing planar ligands, and columnar ligands coordinated between the planar ligands, and
- the at least two organometallic complexes form an inter-penetrating structure such that one apex portion of a unit cell of one of the organometallic complexes is positioned in a space inside one unit cell of the other organometallic complex.

**9.** The gas separation method according to claim 8, further including, after the adsorption step, a desorption step for performing decompression, heating, circulation of the purge gas that does not include the solvent gas, or a combination of these such that the adsorbed solvent gas is desorbed from the gas separation agent.

**10.** A gas separation agent for separating a solvent gas having oxygen atoms from a gas including a hydrocarbon gas, the gas separation agent comprising

- at least two organometallic complexes having a three-dimensional lattice structure defined by opposing metal-containing planar ligands, and columnar ligands coordinated between the planar ligands, wherein
- the at least two organometallic complexes form an inter-penetrating structure such that one apex portion of a unit cell of one of the organometallic complexes is positioned in a space inside one unit cell of the other organometallic complex.

[FIG. 1]

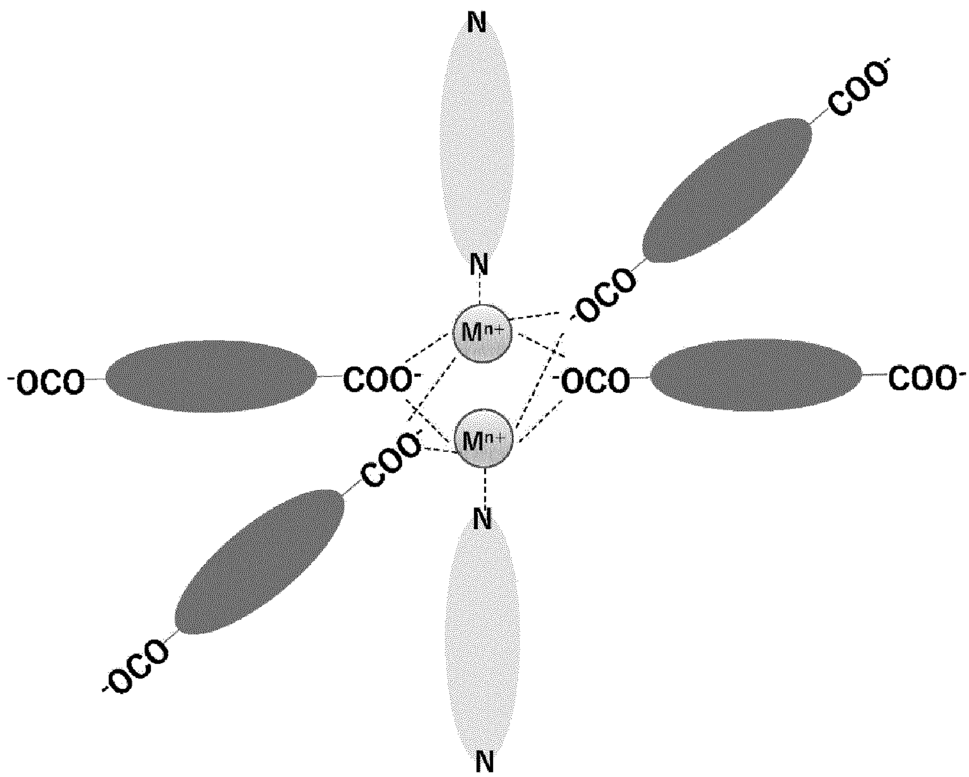


[FIG. 2]

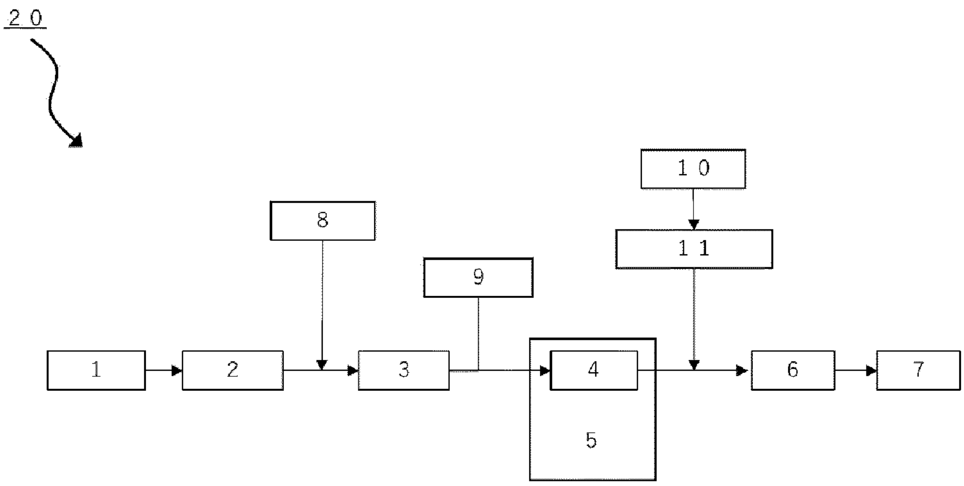

[FIG. 3]

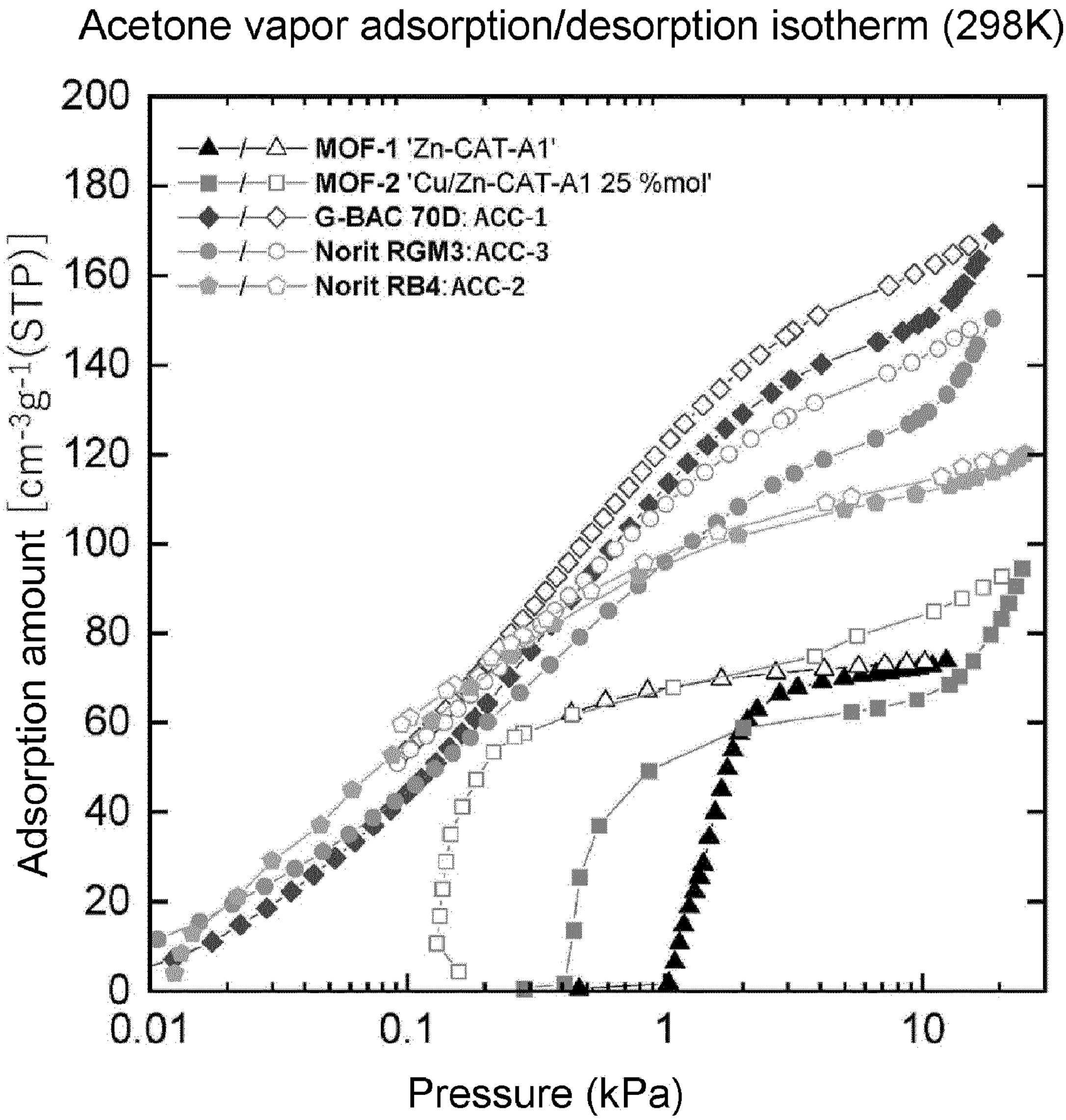
Acetone vapor adsorption/desorption isotherm (298K)
MOF-1 'Zn-CAT-A1'
MOF-2 'Cu/Zn-CAT-A1 25 %mol'
G-BAC 70D: ACC-1
Norit RGM3:ACC-3
Norit RB4:ACC-2
Adsorption amount [cm-3g-1(STP)]
Pressure (kPa)
0
20
40
60
80
100
120
140
160
180
200
0.01
0.1
1
10

[FIG. 4]

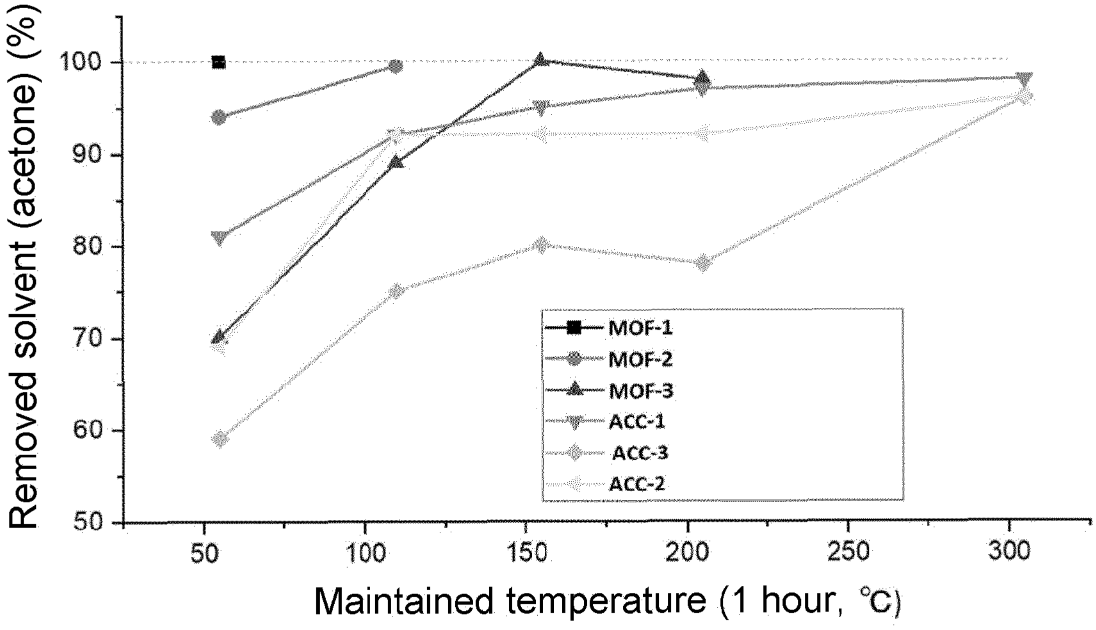
Removed solvent (acetone) (%)
100
90
80
70
60
50
50
100
150
200
250
300
Maintained temperature (1 hour, ℃)
MOF-1
MOF-2
MOF-3
ACC-1
ACC-3
ACC-2

[FIG. 5]

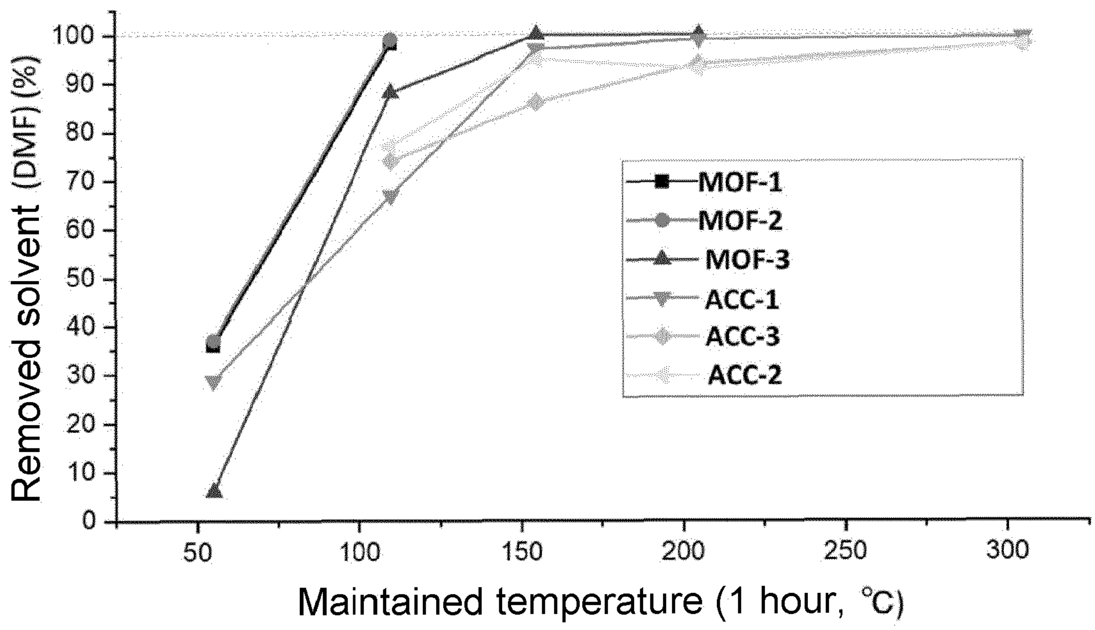
Removed solvent (DMF) (%)
100
90
80
70
60
50
40
30
20
10
0
50
100
150
200
250
300
Maintained temperature (1 hour, ℃)
MOF-1
MOF-2
MOF-3
ACC-1
ACC-3
ACC-2

[FIG. 6]

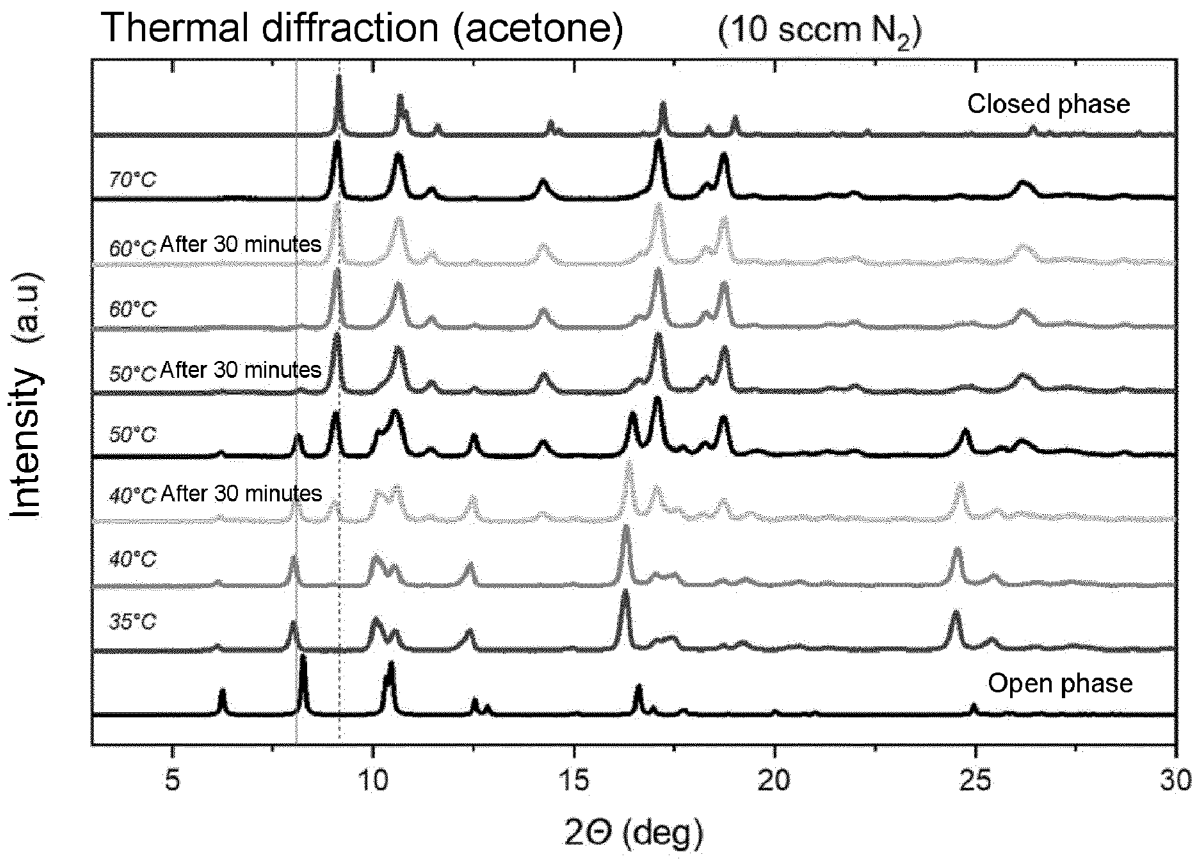
Thermal diffraction (acetone)
(10 sccm $N_2$)
Closed phase
70°C
60°C After 30 minutes
60°C
50°C After 30 minutes
50°C
40°C After 30 minutes
40°C
35°C
Open phase
Intensity (a.u)
5
10
15
20
25
30
2Θ (deg)

[FIG. 7]

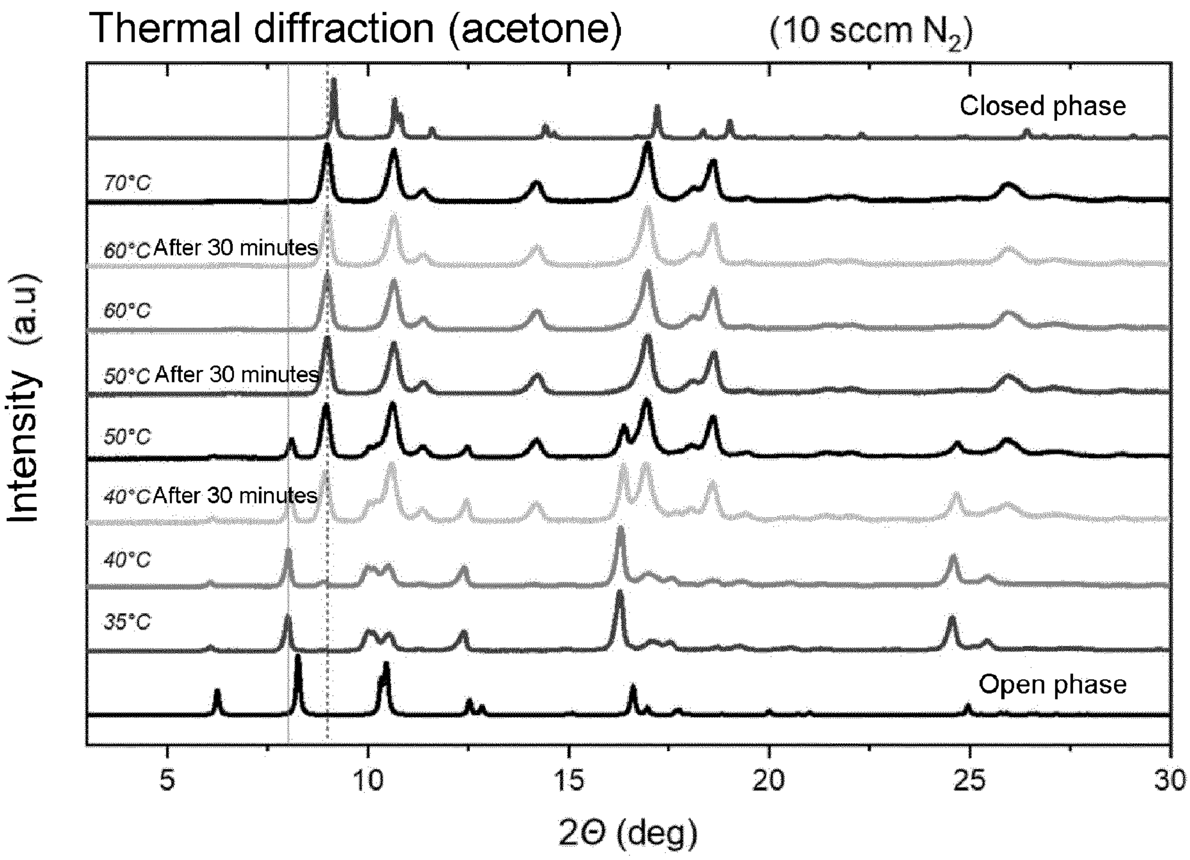
Thermal diffraction (acetone)
(10 sccm $N_2$)
Closed phase
70°C
60°C After 30 minutes
60°C
50°C After 30 minutes
50°C
40°C After 30 minutes
40°C
35°C
Open phase
Intensity (a.u)
5
10
15
20
25
30
2Θ (deg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8398747 B
- WO 2008120160 A
- US 8398747 A
- WO 2008120160 A1